# EUROPEAN PATENT APPLICATION

(11) **EP 4 736 746 A1**
(43) Date of publication of application: **06.05.2026**
(21) Application number: 24913414.9
(22) Date of filing: 26.12.2024
(51) Int. Cl.: A61B 5/00, A61B 7/00, A61B 7/04, G16H 50/20, G06N 3/045

(54) **ELECTRONIC DEVICE AND METHOD FOR CONTROLLING ELECTRONIC DEVICE**

(30) Priority: 27.12.2023 KR 20230193420
(71) Applicant: Samsung Electronics Co., Ltd., Suwon-si, Gyeonggi-do 16677 (KR)
(72) Inventor: MATUSZEWSKI, Mateusz, 00-844 Warszawa (PL); PLES, Anna, 00-844 Warszawa (PL); SIKORSKI, Olaf, 00-844 Warszawa (PL); GORZYNSKI, Kamil, Suwon-si Gyeonggi-do 16677 (KR); TKACZUK, Jakub, 00-844 Warszawa (PL)
(74) Representative: Appleyard Lees IP LLP
(86) International application number: PCT/KR2024/021165
(87) International publication number: WO 2025/143807

(57) **Abstract**

An electronic device is provided. The electronic device includes a microphone, memory storing one or more computer programs, and one or more processors communicatively coupled to the microphone, and the memory, wherein the one or more computer programs include computer-executable instructions that, when executed by the one or more processors individually or collectively, cause the electronic device to store registration information on breathing sounds of a plurality of users in the memory, based on receiving an audio signal through the microphone, obtain information on a breathing sound of a user based on the audio signal, compare the information on the breathing sound with the registration information, identify at least one user corresponding to the information on the breathing sound among the plurality of users, and based on identifying the at least one user, obtain an analysis result for the sleeping states of each of the at least one user based on information corresponding to each of the at least one user in the information on the breathing sound.

## Description

### [Technical Field]

The disclosure relates to an electronic device and a controlling method of the electronic device. More particularly, the disclosure relates to an electronic device that can analyze a breathing sound of a user, and a controlling method thereof.

### [Background Art]

Recently, along with development of fields such as wearable devices, artificial intelligence, the Internet of Things (IoT), etc., development of a technology enabling effective management of a user's health by analyzing a sleeping state of the user is accelerating.

In particular, a technology of analyzing a sleeping state of a user by analyzing a breathing sound that is generated according to breathing during the user's sleep has advantages which are that it can provide information on the sleeping state of the user without interfering with the user's sleep by a non-contact method, and does not need an additional sensor of a high price, etc.

However, regarding such a conventional technology, a limitation is being pointed out, which is that it is difficult to clearly distinguish breathing sounds of a plurality of users in case the plurality of users are sleeping together in a space, and accordingly, it is also difficult to correctly analyze the sleeping states of each of the plurality of users.

The above information is presented as background information only to assist with an understanding of the disclosure. No determination has been made, and no assertion is made, as to whether any of the above might be applicable as prior art with regard to the disclosure.

### [Disclosure]

### [Technical Solution]

Aspects of the disclosure are to address at least the above-mentioned problems and/or disadvantages and to provide at least the advantages described below. Accordingly, an aspect of the disclosure is to provide an electronic device that can clearly distinguish breathing sounds of a plurality of users, and correctly analyze the sleeping states of each of the plurality of users, and a controlling method thereof.

Additional aspects will be set forth in part in the description which follows and, in part, will be apparent from the description, or may be learned by practice of the presented embodiments.

In accordance with an aspect of the disclosure, an electronic device is provided. The electronic device includes a microphone, memory storing one or more computer programs, and one or more processors communicatively coupled to the microphone, and the memory, wherein the one or more computer programs include computer-executable instructions that, when executed by the one or more processors individually or collectively, cause the electronic device to store registration information on breathing sounds of a plurality of users in the memory, based on receiving an audio signal through the microphone, obtain information on a breathing sound of a user based on the audio signal, compare the information on the breathing sound with the registration information, identify at least one user corresponding to the information on the breathing sound among the plurality of users, and based on identifying the at least one user, obtain an analysis result for the sleeping states of each of the at least one user based on information corresponding to each of the at least one user in the information on the breathing sound.

Meanwhile, the one or more processors, based on receiving the audio signal, identify a plurality of segments corresponding to the breathing sound of the user in the audio signal, obtain a plurality of first embedding vectors corresponding to each of the plurality of segments, and based on comparing each of the plurality of first embedding vectors with a plurality of second embedding vectors corresponding to the registration information, identify the at least one user.

Meanwhile, the one or more processors, based on obtaining the plurality of first embedding vectors, identify distances between the locations of each of the plurality of first embedding vectors and the centroid location of embedding vectors corresponding to a first user among the plurality of second embedding vectors in a latent space, and based on the identified distances being smaller than a predetermined threshold distance, identify the first user as the at least one user.

Meanwhile, the one or more processors obtain information on the plurality of segments by inputting the audio signal into a first neural network model trained to distinguish a breathing sound of a user included in an audio signal, obtain the plurality of first embedding vectors by inputting the plurality of segments into a second neural network model trained to convert an input segment into an embedding vector, and obtain the analysis result by inputting the information corresponding to each of the at least one user into a third neural network model trained to identify a sleeping state of a user corresponding to a breathing sound.

Meanwhile, the analysis result includes at least one of information on whether the at least one user is sleeping, information on the sleep quality of the at least one user, or information on the health of the at least one user.

Meanwhile, the electronic device further includes a communicator, and the one or more processors, based on the analysis result indicating that the at least one user is sleeping, obtain a control signal for controlling an external device, and control the communicator to transmit the control signal to the external device.

Meanwhile, the one or more processors, based on identifying the at least one user, update the registration information based on the information on the breathing sound.

Meanwhile, the electronic device further includes a display, and the one or more processors, based on the at least one user not being identified, control the display to display a user interface, and based on receiving a user input for registering the information on the breathing sound through the user interface, add the information on the breathing sound to the registration information.

Meanwhile, the electronic device further includes a sensor, and the one or more processors, based on identifying the at least one user, obtain biometric information of the at least one user through the sensor, and obtain the analysis result for the sleeping states of each of the at least one user based on the information corresponding to each of the at least one user and the biometric information.

In accordance with another aspect of the disclosure, a method performed by an electronic device is provided. The method includes storing registration information on breathing sounds of a plurality of users, based on receiving an audio signal, obtaining information on a breathing sound of a user based on the audio signal, comparing the information on the breathing sound with the registration information on breathing sounds of a plurality of users, and identifying at least one user corresponding to the information on the breathing sound among the plurality of users, and based on identifying the at least one user, obtaining an analysis result for sleeping states of each of the at least one user based on information corresponding to each of the at least one user in the information on the breathing sound.

Meanwhile, the step of obtaining the information on the breathing sound includes the steps of, based on receiving the audio signal, identifying a plurality of segments corresponding to the breathing sound of the user in the audio signal, and obtaining a plurality of first embedding vectors corresponding to each of the plurality of segments, and the step of identifying the at least one user includes the step of, based on comparing each of the plurality of first embedding vectors with a plurality of second embedding vectors corresponding to the registration information, identifying the at least one user.

Meanwhile, the step of identifying the at least one user includes the steps of, based on obtaining the plurality of first embedding vectors, identifying distances between the locations of each of the plurality of first embedding vectors and the centroid location of embedding vectors corresponding to a first user among the plurality of second embedding vectors in a latent space, and based on the identified distances being smaller than a predetermined threshold distance, identifying the first user as the at least one user.

Meanwhile, the step of obtaining the information on the breathing sound includes the steps of obtaining information on the plurality of segments by inputting the audio signal into a first neural network model trained to distinguish a breathing sound of a user included in an audio signal, and obtaining the plurality of first embedding vectors by inputting the plurality of segments into a second neural network model trained to convert an input segment into an embedding vector, and the step of obtaining the analysis result includes the step of obtaining the analysis result by inputting the information corresponding to each of the at least one user into a third neural network model trained to identify a sleeping state of a user corresponding to a breathing sound.

Meanwhile, the analysis result includes at least one of information on whether the at least one user is sleeping, information on the sleep quality of the at least one user, or information on the health of the at least one user.

Meanwhile, the method further includes the steps of, based on the analysis result indicating that the at least one user is sleeping, obtaining a control signal for controlling an external device, and transmitting the control signal to the external device.

Meanwhile, the method further includes the step of, based on identifying the at least one user, updating the registration information based on the information on the breathing sound.

Meanwhile, the method further includes the steps of, based on the at least one user not being identified, displaying a user interface, and based on receiving a user input for registering the information on the breathing sound through the user interface, adding the information on the breathing sound to the registration information.

Meanwhile, the method further includes the steps of, based on identifying the at least one user, obtaining biometric information of the at least one user, and obtaining the analysis result for the sleeping states of each of the at least one user based on the information corresponding to each of the at least one user and the biometric information.

In accordance with another aspect of the disclosure for, one or more non-transitory computer readable storage media storing one or more computer programs including computer-executable instructions that, when executed by one or more processors of an electronic device individually or collectively, cause the electronic device to perform operations are provided. The operations include storing registration information on breathing sounds of a plurality of users, based on receiving an audio signal, obtaining information on a breathing sound of a user based on the audio signal, comparing the information on the breathing sound with registration information on breathing sounds of a plurality of users, identifying at least one user corresponding to the information on the breathing sound among the plurality of users, and based on identifying the at least one user, obtaining an analysis result for the sleeping states of each of the at least one user based on information corresponding to each of the at least one user in the information on the breathing sound.

Other aspects, advantages, and salient features of the disclosure will become apparent to those skilled in the art from the following detailed description, which, taken in conjunction with the annexed drawings, discloses various embodiments of the disclosure.

### [Description of Drawings]

The above and other aspects, features, and advantages of certain embodiments of the disclosure will be more apparent from the following description taken in conjunction with the accompanying drawings, in which:
FIG. 1 is a block diagram schematically illustrating a configuration of an electronic device according to an embodiment of the disclosure;
FIG. 2 is a block diagram schematically illustrating a plurality of modules according to an embodiment of the disclosure;
FIG. 3 is a diagram illustrating an audio signal and information on breathing sounds of a plurality of users according to an embodiment of the disclosure;
FIG. 4 is a diagram for illustrating a process of identifying a user according to an embodiment of the disclosure;
FIG. 5 is a block diagram illustrating in detail a configuration of an electronic device according to an embodiment of the disclosure;
FIG. 6 is a block diagram illustrating in detail a plurality of modules according to an embodiment of the disclosure; and
FIG. 7 is a flow chart schematically illustrating a controlling method of an electronic device according to an embodiment of the disclosure.

The same reference numerals are used to represent the same elements throughout the drawings.

### [Mode for Invention]

The following description with reference to the accompanying drawings is provided to assist in a comprehensive understanding of various embodiments of the disclosure as defined by the claims and their equivalents. It includes various specific details to assist in that understanding but these are to be regarded as merely exemplary Accordingly, those of ordinary skill in the art will recognize that various changes and modifications of the various embodiments described herein can be made without departing from the scope and spirit of the disclosure. In addition, descriptions of well-known functions and constructions may be omitted for clarity and conciseness.

The terms and words used in the following description and claims are not limited to the bibliographical meanings, but, are merely used by the inventor to enable a clear and consistent understanding of the disclosure. Accordingly, it should be apparent to those skilled in the art that the following description of various embodiments of the disclosure is provided for illustration purpose only and not for the purpose of limiting the disclosure as defined by the appended claims and their equivalents.

It is to be understood that the singular forms "a," "an," and "the" include plural referents unless the context clearly dictates otherwise. Thus, for example, reference to "a component surface" includes reference to one or more of such surfaces.

Also, in case it is determined that in describing the disclosure, detailed explanation of related known functions or features may unnecessarily confuse the gist of the disclosure, the detailed explanation will be omitted.

In addition, the embodiments below may be modified in various different forms, and the scope of the technical idea of the disclosure is not limited to the embodiments below. Rather, these embodiments are provided to make the disclosure more sufficient and complete, and to fully convey the technical idea of the disclosure to those skilled in the art.

Further, the terms used in the disclosure are used only to explain specific embodiments, and are not intended to limit the scope of the disclosure. In addition, singular expressions include plural expressions, unless defined obviously differently in the context.

Also, in the disclosure, expressions such as "have," "may have," "include," and "may include" denote the existence of such characteristics (e.g.: elements such as numbers, functions, operations, and components), and do not exclude the existence of additional characteristics.

In addition, in the disclosure, the expressions "A or B," "at least one of A and/or B," or "one or more of A and/or B" and the like may include all possible combinations of the listed items. For example, "A or B," "at least one of A and B," or "at least one of A or B" may refer to all of the following cases: (1) including at least one A, (2) including at least one B, or (3) including at least one A and at least one B.

Further, the expressions "first," "second," and the like used in the disclosure may describe various elements regardless of any order and/or degree of importance. Also, such expressions are used only to distinguish one element from another element, and are not intended to limit the elements.

Meanwhile, the description in the disclosure that one element (e.g.: a first element) is "(operatively or communicatively) coupled with/to" or "connected to" another element (e.g.: a second element) should be interpreted to include both the case where the one element is directly coupled to the another element, and the case where the one element is coupled to the another element through still another element (e.g.: a third element).

In contrast, the description that one element (e.g.: a first element) is "directly coupled" or "directly connected" to another element (e.g.: a second element) can be interpreted to mean that still another element (e.g.: a third element) does not exist between the one element and the another element.

Also, the expression "configured to" used in the disclosure may be interchangeably used with other expressions such as "suitable for," "having the capacity to," "designed to," "adapted to," "made to," and "capable of," depending on cases. Meanwhile, the term "configured to" may not necessarily mean that a device is "specifically designed to" in terms of hardware.

Instead, under some circumstances, the expression "a device configured to" may mean that the device "is capable of' performing an operation together with another device or component. For example, the phrase "a processor configured to perform A, B, and C" may mean a dedicated processor (e.g.: an embedded processor) for performing the corresponding operations, or a generic-purpose processor (e.g.: a CPU or an application processor) that can perform the corresponding operations by executing one or more software programs stored in a memory device.

Also, in the embodiments of the disclosure, 'a module' or 'a part' may perform at least one function or operation, and may be implemented as hardware or software, or as a combination of hardware and software. In addition, a plurality of 'modules' or 'parts' may be integrated into at least one module and implemented as at least one processor, excluding 'a module' or 'a part' that needs to be implemented as specific hardware.

Meanwhile, various elements and areas in drawings were illustrated schematically. Accordingly, the technical idea of the disclosure is not limited by the relative sizes or intervals illustrated in the accompanying drawings.

Hereinafter, embodiments according to the disclosure will be described in detail with reference to the accompanying drawings, such that those having ordinary skill in the art to which the disclosure belongs can easily carry out the disclosure.

It should be appreciated that the blocks in each flowchart and combinations of the flowcharts may be performed by one or more computer programs which include instructions. The entirety of the one or more computer programs may be stored in a single memory device or the one or more computer programs may be divided with different portions stored in different multiple memory devices.

Any of the functions or operations described herein can be processed by one processor or a combination of processors. The one processor or the combination of processors is circuitry performing processing and includes circuitry like an application processor (AP, e.g. a central processing unit (CPU)), a communication processor (CP, e.g., a modem), a graphics processing unit (GPU), a neural processing unit (NPU) (e.g., an artificial intelligence (AI) chip), a Wi-Fi chip, a Bluetooth^{®} chip, a global positioning system (GPS) chip, a near field communication (NFC) chip, connectivity chips, a sensor controller, a touch controller, a fingerprint sensor controller, a display driver integrated circuit (IC), an audio CODEC chip, a universal serial bus (USB) controller, a camera controller, an image processing IC, a microprocessor unit (MPU), a system on chip (SoC), an IC, or the like.

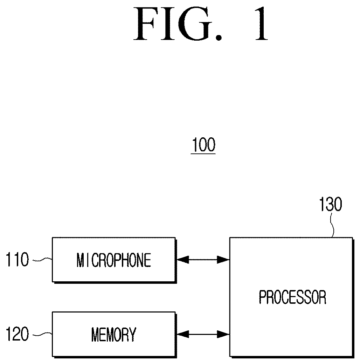

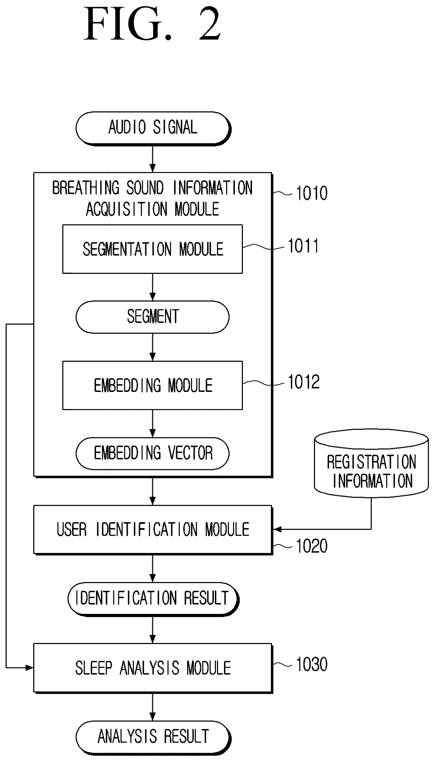

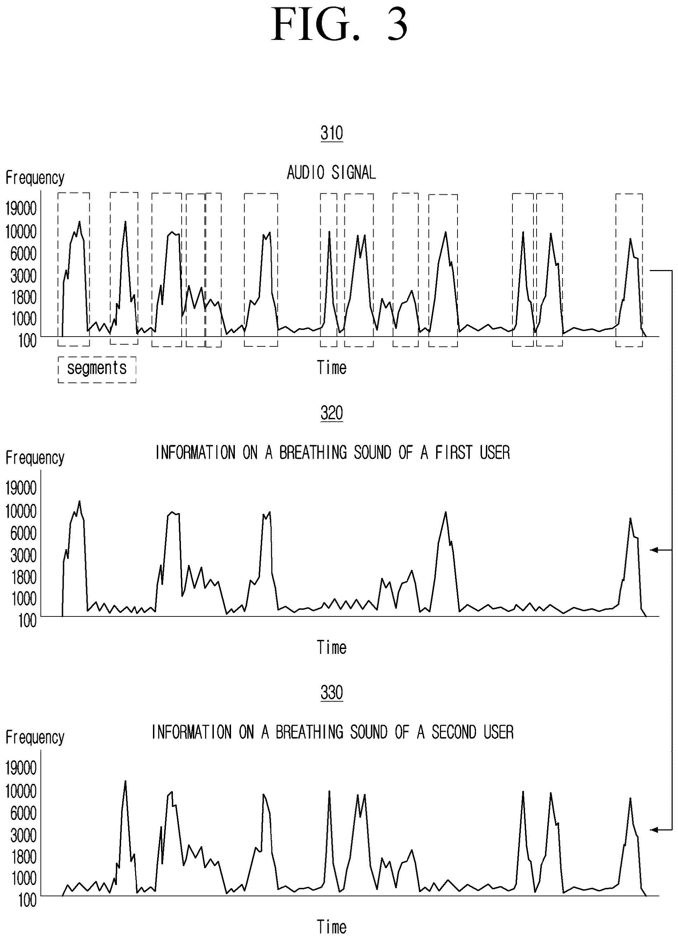

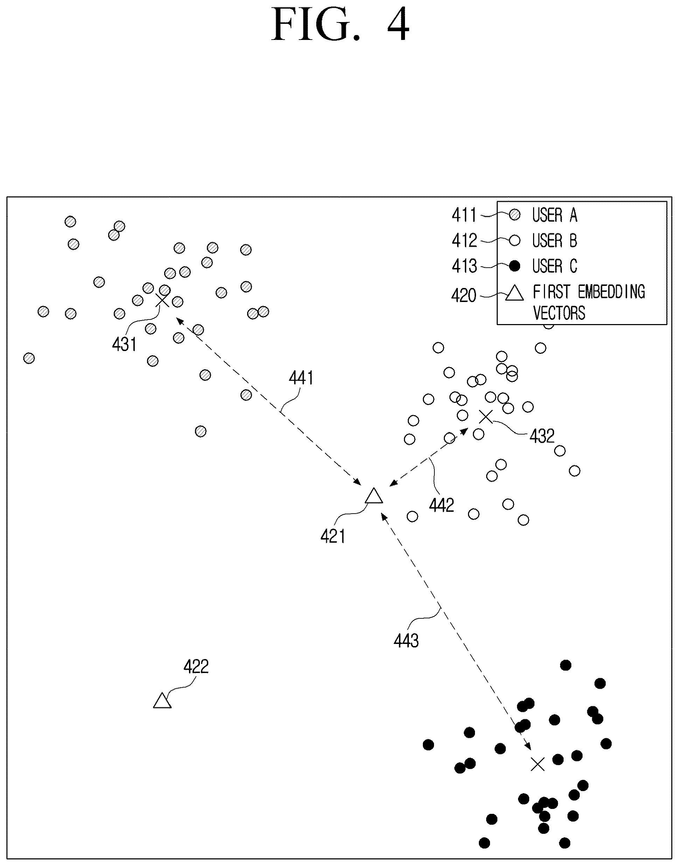

FIG. 1 is a block diagram schematically illustrating a configuration of an electronic device 100 according to an embodiment of the disclosure. FIG. 2 is a block diagram schematically illustrating a plurality of modules according to an embodiment of the disclosure. Also, FIG. 3 is a diagram illustrating an audio signal and information on breathing sounds of a plurality of users according to an embodiment of the disclosure, and FIG. 4 is a diagram for illustrating a process of identifying a user according to an embodiment of the disclosure. Hereinafter, explanation will be described with reference to FIGS. 1 to 4 together.

Referring to FIG. 1, the electronic device 100 according to an embodiment of the disclosure may include a microphone 110, memory 120, and a processor 130.

The microphone 110 may obtain a signal for a sound or a voice generated outside the electronic device 100. Specifically, the microphone 110 may obtain vibration according to a sound or a voice generated outside the electronic device 100, and convert the obtained vibration into an electric signal.

In particular, the microphone 110 according to the disclosure may obtain a voice signal for a user voice generated by the user's utterance. Then, the obtained signal may be converted into a signal of a digital form, and stored in the memory 120. The microphone 110 may include an analog to digital (A/D) converter, and may also operate by being interlocked with an A/D converter located outside the microphone 110.

According to an embodiment, the processor 130 may receive an audio signal through the microphone 110. In particular, 'an audio signal' may be an audio signal that was received through the microphone 110 while at least one user was sleeping, and it may include information on a breathing sound of the user as will be described below. For example, if an audio signal is received through the microphone 110 while a user A and a user B are sleeping, the received audio signal may include information on a breathing sound of the user A and information on a breathing sound of the user B together.

In the memory 120, at least one instruction regarding the electronic device 100 may be stored. Also, in the memory 120, an operating system (O/S) for operating the electronic device 100 may be stored. In addition, in the memory 120, various types of software programs or applications for the electronic device 100 to operate according to the various embodiments of the disclosure may be stored. Further, the memory 120 may include semiconductor memory such as flash memory, etc., or a magnetic storage medium such as a hard disk, etc.

Specifically, in the memory 120, various kinds of software modules for the electronic device 100 to operate according to the various embodiments of the disclosure may be stored, and the processor 130 may control the operations of the electronic device 100 by executing the various kinds of software modules stored in the memory 120. That is, the memory 120 may be accessed by the processor 130, and reading/recording/correction/deletion/update, etc. of data by the processor 130 may be performed.

Meanwhile, in the disclosure, the term memory 120 may be used as a meaning including the memory 120, read only memory (ROM) and random access memory (RAM) inside the processor 130, or a memory card (e.g., a micro secure digital (SD) card, a memory stick) installed on the electronic device 100.

In particular, according to an embodiment, registration information on breathing sounds of a plurality of users may be stored. Here, 'registration information' refers to information stored in the memory 120 as a plurality of users input information on their breathing sounds. That is, the registration information is a term for distinctively specifying information on a breathing sound included in an audio signal that is received through the microphone 110 in real time, and information on a breathing sound that was stored in the memory 120 according to a user's input beforehand.

'Information on a breathing sound' is used as a term for generally referring to information on the characteristics of a breathing sound of a user, and in particular, it may include information on a breathing sound that is generated according to breathing during a user's sleep. Specifically, information on a breathing sound may include various kinds of information such as a frequency characteristic, a pattern of change, etc. of a section corresponding to the breathing sound in an audio signal. Also, information on a breathing sound may include at least one of information on a segment corresponding to the breathing sound, or information on an embedding vector corresponding to the segment.

Also, in the memory 120, various kinds of information/data such as an audio signal, information on a breathing sound of a user, information on a segment corresponding to the breathing sound, information on an embedding vector corresponding to the segment, information on an identification result of the user, an analysis result for a sleeping state, a control signal, data for a neural network model, etc. may be stored.

Other than the above, various kinds of information necessary within a range for achieving the purpose of the disclosure may be stored in the memory 120, and the information stored in the memory 120 may be updated as information is received from an external device or input by a user.

The processor 130 controls the overall operations of the electronic device 100. Specifically, the processor 130 may be connected with the components of the electronic device 100 including the microphone 110 and the memory 120, and control the overall operations of the electronic device 100 by executing at least one instruction stored in the memory 120 as described above.

The processor 130 may be implemented in various ways. For example, the processor 130 may be implemented as at least one of an application specific integrated circuit (ASIC), an embedded processor, a microprocessor, a hardware control logic, a hardware finite state machine (FSM), or a digital signal processor (DSP). Meanwhile, in the disclosure, the term processor may be used as a meaning including a central processing unit (CPU), a graphic processing unit (GPU), and a microprocessor unit (MPU), etc.

Various operations of the processor 130 may be implemented through a plurality of modules. Specifically, data for the plurality of modules may be stored in the memory 120. Further, the processor 130 may load the data for the plurality of modules stored in the memory 120 on the memory 120 or the memory 120 included in the processor 130, and implement the various embodiments according to the disclosure by using the plurality of modules. The plurality of modules may not only be implemented as software modules, but also be implemented as hardware modules.

Referring to FIG. 2, the plurality of modules may include a breathing sound information acquisition module 1010, a user identification module 1020, and a sleep analysis module 1030. Also, the breathing sound information acquisition module 1010 may include a segmentation module 1011 and an embedding module 1012.

In particular, according to an embodiment, if an audio signal is received through the microphone 110, the processor 130 may obtain information on a breathing sound of a user based on the audio signal. As the process of obtaining information on a breathing sound may include a segment extraction process and an embedding vector acquisition process, detailed explanation in this regard will be described below.

When an audio signal is received, the processor 130 may identify a plurality of segments corresponding to a breathing sound of a user in the audio signal. As illustrated in FIG. 2, the processor 130 may identify a plurality of segments corresponding to a breathing sound of a user in an audio signal by inputting the audio signal into the segmentation module 1011.

The processor 130 may identify a section including a characteristic corresponding to the breathing sound of the user in the audio signal, and extract the identified section as a segment. That is, 'a segment' refers to a result of extracting each section including characteristics corresponding to the breathing sound of the user in the audio signal, and it may be replaced by terms such as 'a slice,' 'a portion,' 'a subset,' etc. For example, characteristics corresponding to a breathing sound may not only include a user's inhalation and exhalation, but also include a snoring sound, a sound indicating a symptom of apnea, etc.

The processor 130 may identify a segment including a characteristic corresponding to a breathing sound of a user in an audio signal by using various technics such as analysis of frequency characteristics of the audio signal, analysis of energy and amplitude, visualization of a waveform, etc. Also, the processor 130 may obtain information on a plurality of segments by inputting the audio signal into a first neural network model trained to distinguish a breathing sound of a user included in an audio signal.

The image 310 in FIG. 3 indicates a plurality of segments corresponding to a breathing sound of a user with a box consisting of dotted lines together with an audio signal. As illustrated in FIG. 3, the sizes of each of the plurality of segments do not necessarily have to be identical.

When a plurality of segments are identified, the processor 130 may obtain a plurality of first embedding vectors corresponding to each of the plurality of segments. As illustrated in FIG. 2, when information on the plurality of segments is obtained through the segmentation module 1011, the processor 130 may obtain a plurality of first embedding vectors corresponding to each of the plurality of segments by inputting the information on the plurality of segments into the embedding module 1012.

'An embedding vector' generally refers to a result of digitizing each of the plurality of segments such that the characteristics of each of the plurality of segments can be distinguished, and 'the first embedding vector' is an embedding vector corresponding to a segment extracted from an audio signal received through the microphone 110, and is distinguished from 'a second embedding vector' which means an embedding vector corresponding to/included in the registration information.

Hereinafter, explanation will be described by using the term embedding 'vector' based on the premise that the characteristics of each segment extracted from an audio signal are digitized to vectors of a fixed dimension, but depending on embodiments, each of the plurality of segments may be converted into a form of a real number, a matrix, or a tensor.

Specifically, the processor 130 may obtain a plurality of first embedding vectors corresponding to each of the plurality of segments by using technics such as Mel-Frequency Cepstral Coefficients (MFCC), spectrogram-based embeddings, etc. Also, the processor 130 may obtain the first embedding vectors by inputting the plurality of segments into a second neural network model trained to convert information on the input segments into embedding vectors.

The processor 130 may compare information on a breathing sound with the registration information, and identify at least one user corresponding to the information on the breathing sound among a plurality of users. As illustrated in FIG. 2, if information on a breathing sound (specifically, embedding vectors) is obtained through the breathing sound information acquisition module 1010, the processor 130 may input the information on the breathing sound and the registration information stored in the memory 120 into the user identification module 1020, and identify at least one user corresponding to the information on the breathing sound among a plurality of users.

Specifically, based on comparing each of the plurality of first embedding vectors with a plurality of second embedding vectors corresponding to the registration information, the processor 130 may identify at least one user.

According to an embodiment, when the plurality of first embedding vectors are obtained, the processor 130 may identify distances between the locations of each of the plurality of first embedding vectors and the centroid location of embedding vectors corresponding to a first user among the plurality of second embedding vectors in a latent space.

Here, 'a latent space' is an abstract space for expressing characteristics of the embedding vectors, and each axis of the latent space may indicate one of the characteristics of the embedding vectors. Accordingly, the locations of the embedding vectors in the latent space may correspond to the characteristics of the embedding vectors. As an example, FIG. 4 illustrates the locations of each of the plurality of first embedding vectors and the locations of the plurality of second embedding vectors in a latent space.

Referring to FIG. 4, the locations of the first embedding vectors were indicated as triangles, and the locations of the second embedding vectors were indicated as circles. Specifically, the second embedding vectors in FIG. 4 include embedding vectors 411 corresponding to the user A, embedding vectors 412 corresponding to the user B, and embedding vectors 413 corresponding to the user C. In other words, the second embedding vectors in FIG. 4 refer to embedding vectors that were registered based on the user A, the user B, and the user C inputting information on their breathing sounds.

As the breathing sounds of each of the user A, the user B, and the user C indicate characteristics different from one another, among the second embedding vectors, the embedding vectors 411 corresponding to the user A, the embedding vectors 412 corresponding to the user B, and the embedding vectors 413 corresponding to the user C have locations distinguished from one another in a latent space.

Meanwhile, the first embedding vectors 420 in FIG. 4 include the embedding vector 421 corresponding to the first segment and the embedding vector 422 corresponding to the second segment extracted from the same audio signal.

When the embedding vector 421 corresponding to the first segment is obtained, the processor 130 may map the embedding vector 421 corresponding to the first segment to a latent space, and calculate a distance between the embedding vector 421 corresponding to the first segment and the embedding vectors 411 corresponding to the user A. Specifically, the processor 130 may calculate only a distance between the location of the embedding vector 421 corresponding to the first segment and the centroid location of the embedding vectors 411 corresponding to the user A.

Here, 'the centroid location' refers to a location that can represent the embedding vectors. The centroid location may be determined based on an average value of the embedding vectors, or a median value of the embedding vectors, and may also be determined based on Principal Component Analysis (PCA) using principal component analysis for the embedding vectors, a Gaussian Mixture Model (GMM) using an average value of a result of modeling distribution of the embedding vectors with a Gaussian Mixture Model, etc.

The processor 130 may determine relevance between the plurality of first embedding vectors and the first user based on the identified distances as described above, and accordingly, identify a user corresponding to information on a breathing sound included in an audio signal.

According to an embodiment, if the distances between the locations of each of the plurality of first embedding vectors and the centroid location of embedding vectors corresponding to the first user among the plurality of second embedding vectors are smaller than a predetermined threshold distance, the processor 130 may identify the first user as the at least one user corresponding to information on a breathing sound included in an audio signal.

In the example of FIG. 4, if the distance between the location of the embedding vector 421 corresponding to the first segment and the centroid location 431 of the embedding vectors 411 corresponding to the user A is smaller than the threshold distance, the processor 130 may determine that the relevance between the embedding vector 421 corresponding to the first segment and the embedding vectors 411 corresponding to the user A is high, and accordingly, identify that a user corresponding to information on a breathing sound included in an audio signal is the registered user A.

In contrast, in the example of FIG. 4, if the distance between the location of the embedding vector 421 corresponding to the first segment and the centroid location 431 of the embedding vectors 411 corresponding to the user A is greater than or equal to the threshold distance, the processor 130 may determine that the relevance between the embedding vector 421 corresponding to the first segment and the embedding vectors 411 corresponding to the user A is low, and accordingly, identify that a user corresponding to information on a breathing sound included in an audio signal is not the registered user A.

In the above, only a processing process for the embedding vector 421 corresponding to the first segment was explained, but the processing process as described above can be performed for the entire embedding vectors corresponding to a plurality of segments included in an audio signal, and as a result, it may be identified which user each of the plurality of entire segments included in the audio signal corresponds to.

In the above, an embodiment of calculating distances between the locations of each of the plurality of first embedding vectors and the centroid location of embedding vectors corresponding to the first user among the plurality of second embedding vectors was explained, but the disclosure is not limited thereto. For example, the processor 130 may calculate all the distances between the location of the embedding vector 421 corresponding to the first segment and the locations of each of the embedding vectors 411 corresponding to the user A, and identify at least one user corresponding to information on a breathing sound included in an audio signal based on all of the calculated distances.

According to an embodiment, the processor 130 may identify a user corresponding to information on a breathing sound included in an audio signal based on the distances between the locations of each of the plurality of first embedding vectors and each of the centroid locations of the second embedding vectors corresponding to the plurality of users.

In the example of FIG. 4, the processor 130 may identify a user corresponding to information on a breathing sound included in an audio signal by calculating each of a first distance 441 between the location of the embedding vector 421 corresponding to the first segment and the centroid location of the embedding vectors 411 corresponding to the user A, a second distance 442 between the location of the embedding vector 421 corresponding to the first segment and the centroid location 432 of the embedding vectors 412 corresponding to the user B, and a third distance 443 between the location of the embedding vector 421 corresponding to the first segment and the centroid location 433 of the embedding vectors 413 corresponding to the user C, and comparing the calculated first distance, second distance, and third distance.

For example, as in the example of FIG. 4, in case the second distance is shorter compared to the first distance and the third distance, the processor 130 may identify the user B corresponding to the second distance as the user corresponding to information on a breathing sound included in an audio signal.

Meanwhile, the first embedding vector of which distances with all centroid locations of the second embedding vectors are greater than or equal to the threshold distance among the plurality of first embedding vectors may be regarded as an abnormal sample, and may be excluded from a user identification process.

When at least one user is identified, the processor 130 may obtain an analysis result for the sleeping states of each of the at least one user based on information corresponding to each of the at least one user in the information on the breathing sound included in the audio signal. As illustrated in FIG. 2, the processor 130 may obtain an analysis result for the sleeping states of each of the at least one user by inputting the identification result obtained through the user module and the information on the breathing sound obtained through the breathing sound information acquisition module 1010 into the sleep analysis module 1030.

The processor 130 may distinctively extract information on a breathing sound of the first user and information on a breathing sound of the second user from the information on the breathing sound included in the audio signal. Then, the processor 130 may obtain an analysis result for the sleeping state of the first user by using only the information on the breathing sound of the first user, and obtain an analysis result for the sleeping state of the second user by using only the information on the breathing sound of the second user.

Referring to FIG. 3, the processor 130 may identify a plurality of segments (a box consisting of dotted lines) corresponding to a breathing sound of a user in an audio signal indicated by the image 310, and identify first segments corresponding to the first user and second segments corresponding to the second user among the plurality of segments by comparing the plurality of first embedding vectors corresponding to each of the plurality of segments with the plurality of second embedding vectors corresponding to the registration information. Accordingly, the processor 130 may obtain information on a breathing sound of the first user including the first segments as indicated by the image 320, and obtain information on a breathing sound of the second user including the second segments as indicated by the image 330.

The analysis result may include at least one of information on whether the at least one user is sleeping, information on the sleep quality of the at least one user, or information on the health of the at least one user. For example, the analysis result may include information on the user's sleeping time, the sleeping stage (e.g.: awake, light sleep, deep sleep, rapid eye movement (REM) sleep), the sleep quality, etc. Also, the analysis result may include information on the user's health such as the user's stress, the user's breathing state, etc.

The processor 130 may obtain an analysis result for the sleeping states of each of the at least one user by using the cycles, the number of times, and the frequencies of breathing sounds corresponding to each of the at least one user. Also, the processor 130 may obtain an analysis result by inputting the information corresponding to each of the at least one user in the information on the breathing sound included in the audio signal into a third neural network model trained to identify a sleeping state of a user corresponding to a breathing sound.

According to the embodiments described above, the electronic device 100 can clearly distinguish breathing sounds of a plurality of users by using only information on the breathing sounds of the users by a non-contact method, and accordingly, clearly analyze the sleeping states of each of the plurality of users.

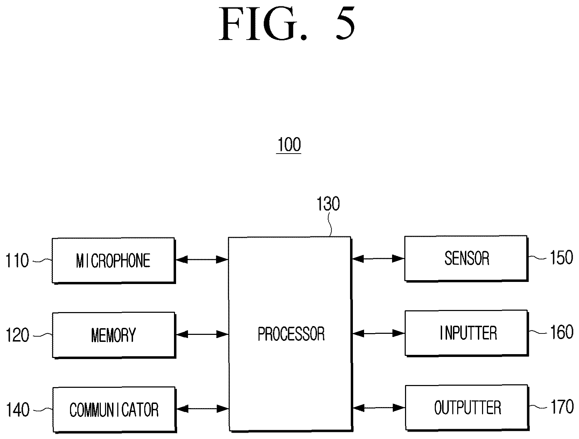

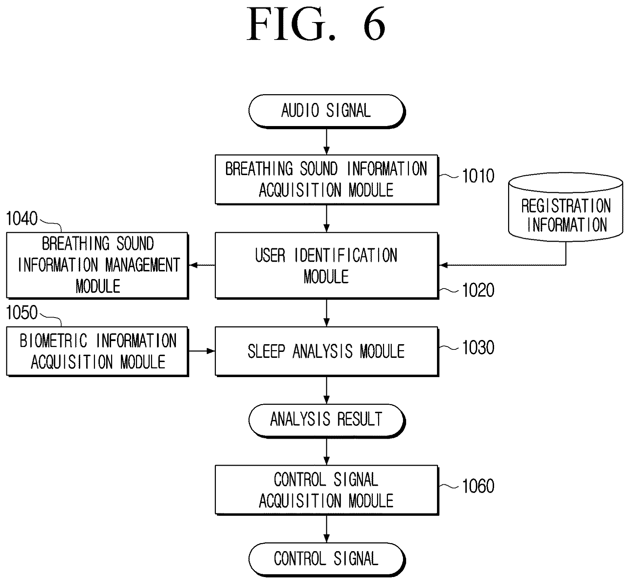

FIG. 5 is a block diagram illustrating in detail a configuration of the electronic device 100 according to an embodiment of the disclosure, and FIG. 6 is a block diagram illustrating in detail a plurality of modules according to an embodiment of the disclosure.

Referring to FIG. 5, the electronic device 100 may further include a communicator 140, a sensor 150, an inputter 160, and an outputter 170 as well as the microphone 110, the memory 120, and the processor 130. However, the components as illustrated in FIGS. 1 and 5 are merely exemplary ones, and in carrying out the disclosure, it is obvious that new components can be added in addition to the components as illustrated in FIGS. 1 and 5, or some components can be omitted.

Referring to FIG. 6, the plurality of modules may further include a breathing sound information management module 1040, a biometric information acquisition module 1050, and a control signal acquisition module 1060 as well as the breathing sound information acquisition module 1010, the user identification module 1020, and the sleep analysis module 1030. However, the components of the plurality of modules as illustrated in FIGS. 2 and 6 are also merely exemplary ones.

The communicator 140 (i.e., transceiver) may include circuitry, and perform communication with an external device. Specifically, the processor 130 may receive various kinds of data or information from an external device connected through the communicator 140, or transmit various kinds of data or information to an external device.

The communicator 140 may include at least one of a Wi-Fi module, a Bluetooth module, a wireless communication module, an NFC module, or an ultra-wide band (UWB) module. Specifically, each of a Wi-Fi module and a Bluetooth module may perform communication by a Wi-Fi method and a Bluetooth method, respectively. In the case of using a Wi-Fi module or a Bluetooth module, various kinds of connection information such as an SSID, etc. is transmitted and received first, and connection of communication is performed by using the information, and various kinds of information can be transmitted and received thereafter.

Also, a wireless communication module may perform communication according to various communication protocols such as IEEE, Zigbee, 3rd Generation (3G), 3rd Generation Partnership Project (3GPP), Long Term Evolution (LTE), 5th Generation (5G), etc. In addition, an NFC module may perform communication by a near field communication (NFC) method using a 13.56MHz band among various RF-ID frequency bands such as 135kHz, 13.56MHz, 433MHz, 860 - 960MHz, 2.45GHz, etc. Further, a UWB module can correctly measure a Time of Arrival (ToA) which is the time that a pulse reaches a target, and an Angle of Arrival (AoA) which is a pulse arrival angle in a transmission device through communication between UWB antennas, and accordingly, the UWB module can perform precise distance and location recognition in an error range of within scores of cm indoors.

In particular, according to an embodiment, if an analysis result for the sleeping states of each of the at least one user indicates that the at least one user is sleeping, the processor 130 may obtain a control signal for controlling an external device, and control the communicator 140 to transmit the control signal to the external device. Referring to FIG. 6, the processor 130 may obtain a control signal for controlling an external device by inputting an analysis result obtained through the sleep analysis module 1030 into the control signal acquisition module 1060.

In other words, as a result of identifying a user corresponding to information on a breathing sound included in an audio signal, if at least one user is identified, and all of the identified at least one user is sleeping, the processor 130 may obtain a control signal for controlling an external device based on the information that all the users are sleeping, and control the communicator 140 to transmit the control signal to the external device. Here, the external device may be a device that was registered as a device constituting the same Internet of Things (IoT) network as the electronic device 100.

For example, based on the information that all the users are sleeping, the processor 130 may obtain a control signal for turning off the power of an illumination device, a speaker device, a television (TV), etc., and control the communicator 140 to transmit the control signal to an external device.

Other than the above, the processor 130 may receive registration information on breathing sounds of a plurality of users, information on a neural network model, etc. through the communicator 140, and control the communicator 140 to transmit an analysis result for the sleeping states of the users to external devices such as the smartphones, the smart watches, etc. of the users.

The sensor 150 may detect various kinds of information inside and outside the electronic device 100. Specifically, the sensor 150 may include at least one of a global positioning system (GPS) sensor 150, a gyro sensor (a gyroscope) 150, an acceleration sensor (an accelerometer) 150, a light detection and ranging (LiDAR) sensor 150, an inertial sensor (an inertial measurement unit (IMU)) 150, or a motion sensor 150. Not only that, the sensor 150 may include various kinds of sensors 150 such as a temperature sensor 150, a humidity sensor 150, an infrared sensor 150, a bio sensor 150, etc.

In particular, the sensor 150 according to the disclosure may include at least one of an image sensor 150 that can obtain images for a user according to time, a motion sensor 150 that can detect a motion of a user, or a biometric sensor 150 that can detect a signal related to biometric information of a user. The biometric sensor 150 may include sensors 150 such as a heartrate sensor 150, a bio impedance sensor 150, a blood pressure sensor 150, etc.

According to an embodiment, when at least one user is identified, the processor 130 may obtain biometric information for the at least one user through the sensor 150. Then, based on the information corresponding to each of the at least one user and the biometric information, the processor 130 may obtain an analysis result for the sleeping states of each of the at least one user.

Referring to FIG. 6, the processor 130 may obtain an analysis result for the sleeping states of the users by inputting the biometric information obtained through the biometric information acquisition module 1050 into the sleep analysis module 1030 together with the information on the breathing sounds of the users obtained through the breathing sound information acquisition module 1010.

For example, in case the electronic device 100 includes the sensor 150, the processor 130 may obtain an analysis result for the sleeping states of each user by using the biometric information such as the information on the blood pressure of the users, the information on the heart rates of the users, etc. together with the information on the breathing sounds of the users.

Meanwhile, various kinds of sensing information such as the movements of the users, the postures of the users, etc. as well as the biometric information may be used in analysis of the sleeping states.

The inputter 160 may include circuitry, and the processor 130 may receive a user instruction for controlling the operation of the electronic device 100 through the inputter 160. Specifically, the inputter 160 may consist of components such as a microphone 110, a camera, and a remote control signal receiver, etc. Also, the inputter 160 may be implemented in a form of being included in the display as a touch screen. In particular, the microphone 110 may receive a voice signal, and convert the received voice signal into an electric signal.

In particular, according to an embodiment, the processor 130 may receive a user input for registering information on a breathing sound of a user, a user input for updating registration information on a breathing sound of a user, etc. through the inputter 160.

The outputter 170 may include circuitry, and the processor 130 may output various functions that can be performed by the electronic device 100 through the outputter 170. Also, the outputter 170 may include at least one of a display, a speaker, or an indicator.

The display may output image data by control by the processor 130. Specifically, the display may output an image stored in advance in the memory 120 by control by the processor 130. In particular, the display according to an embodiment of the disclosure may display a user interface stored in the memory 120.

The display may be implemented as a liquid crystal display (LCD) panel, organic light emitting diodes (OLEDs), etc., and the display may also be implemented as a flexible display, a transparent display, etc. depending on cases. However, the display according to the disclosure is not limited to a specific type.

The speaker may output audio data by control by the processor 130.

The indicator may be turned on by control by the processor 130. Specifically, the indicator may be turned on in various colors according to control by the processor 130. For example, the indicator may be implemented as light emitting diodes (LEDs), a liquid crystal display (LCD) panel, a vacuum fluorescent display (VFD), etc., but is not limited thereto.

In particular, according to an embodiment, if at least one user is not identified as a result of identifying a user corresponding to information on a breathing sound included in an audio signal, the processor 130 may input the identification result into the breathing sound information management module 1040, and perform an operation for adding registration information.

Specifically, if at least one user is not identified as a result of identifying a user corresponding to information on a breathing sound included in an audio signal, the processor 130 may control the display to display a user interface. Here, the user interface may be for requesting to register information on a breathing sound to a user. If a user input for registering information on a breathing sound is received through the user interface, the processor 130 may add the information on the breathing sound to the registration information.

For example, if a user input for initiating registration of information on a breathing sound is received through the user interface, the processor 130 may initiate registration of the information on the breathing sound. Afterwards, the processor 130 may receive an audio signal according to the user's breathing through the microphone 110, and store information on the breathing sound included in the received audio signal as registration information.

The process of registering information on a breathing sound as registration information may include a process of identifying segments in an audio signal, and a process of obtaining embedding vectors corresponding to the segments, as described above. The processor 130 may identify segments wherein the characteristics of the audio signal are clear, and store only embedding vectors corresponding to the clear segments as registration information.

Meanwhile, in the above, an embodiment wherein registration information is added in case at least one user is not identified as a result of identifying a user corresponding to information on a breathing sound included in an audio signal was explained. However, in contrast, in case at least one user is identified as a result of identifying a user corresponding to information on a breathing sound included in an audio signal, the processor 130 may input the identification result into the breathing sound information management module 1040, and perform an operation for updating the registration information.

According to an embodiment, if at least one user is identified as a result of identifying a user corresponding to information on a breathing sound included in an audio signal, the processor 130 may update the registration information based on the information on the breathing sound. Referring to FIG. 6, the processor 130 may obtain the updated registration information by inputting the information on the breathing sound into the breathing sound information management module.

Specifically, if at least one user is identified, the processor 130 may distinctively extract information on a breathing sound of the first user and information on a breathing sound of the second user from information on a breathing sound included in an audio signal. Then, the processor 130 may update the registration information for the first user based on the information on the breathing sound of the first user, and update the registration information for the second user based on the information on the breathing sound of the second user.

For example, updating the registration information may mean adding the first embedding vector corresponding to the first user to the second embedding vector corresponding to the first user, and adding the first embedding vector corresponding to the second user to the second embedding vector corresponding to the second user in a latent space as illustrated in FIG. 4.

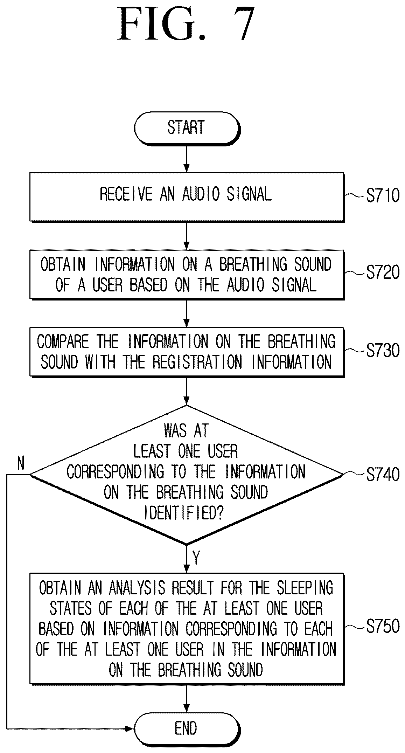

FIG. 7 is a flow chart schematically illustrating a controlling method of the electronic device 100 according to an embodiment of the disclosure.

Referring to FIG. 7, the electronic device 100 may receive an audio signal in operation S710. Specifically, the electronic device 100 may receive an audio signal through the microphone 110 included in the electronic device 100, or receive an audio signal from an external device.

The electronic device 100 may obtain information on a breathing sound of a user based on the audio signal in operation S720. Specifically, the electronic device 100 may identify a plurality of segments corresponding to a breathing sound of a user in the audio signal, and obtain a plurality of first embedding vectors corresponding to each of the plurality of segments.

The electronic device 100 may compare the information on the breathing sound with registration information in operation S730, and identify at least one user corresponding to the information on the breathing sound in operation S740. Here, the registration information on the breathing sounds of the plurality of users may be stored in the memory 120 of the electronic device 100, or received from an external device. Specifically, the electronic device 100 may identify at least one user based on comparing each of the plurality of first embedding vectors with a plurality of second embedding vectors corresponding to the registration information.

If at least one user is identified in operation S740-Y, the electronic device 100 may obtain an analysis result for the sleeping states of each of the at least one user based on information corresponding to each of the at least one user in the information on the breathing sound in operation S750. The analysis result may include at least one of information on whether the at least one user is sleeping, information on the sleep quality of the at least one user, or information on the health of the at least one user.

Meanwhile, if at least one user is not identified in operation S740-N, the electronic device 100 may finish its operation, and provide a user interface for adding registration information. Then, if a user input for registering information on a breathing sound is received through the user interface, the electronic device 100 may add the information on the breathing sound to the registration information.

Meanwhile, the controlling method of the electronic device 100 according to the aforementioned embodiment may be implemented as a program and provided to the electronic device 100. In particular, a program including the controlling method of the electronic device 100 may be provided while being stored in a non-transitory computer readable medium.

Specifically, in a non-transitory computer readable recording medium including a program executing a controlling method of the electronic device 100, the controlling method of the electronic device 100 includes the steps of, based on receiving an audio signal, obtaining information on a breathing sound of a user based on the audio signal, comparing the information on the breathing sound with registration information on breathing sounds of a plurality of users, and identifying at least one user corresponding to the information on the breathing sound among the plurality of users, and based on identifying the at least one user, obtaining an analysis result for the sleeping states of each of the at least one user based on information corresponding to each of the at least one user in the information on the breathing sound.

In the above, a controlling method of the electronic device 100, and a computer readable recording medium including a program executing the controlling method of the electronic device 100 were explained briefly, but this is just for omitting overlapping explanation, and the various embodiments regarding the electronic device 100 can obviously be applied to the controlling method of the electronic device 100, and the computer readable recording medium including a program executing the controlling method of the electronic device 100.

Functions related to artificial intelligence according to the disclosure are operated through the processor 130 and the memory 120 of the electronic device 100.

The processor 130 may consist of one or a plurality of processors 130. Here, the one or plurality of processors 130 may include at least one of a central processing unit (CPU), a graphic processing unit (GPU), or a neural processing unit (NPU), but the processors are not limited to the aforementioned examples of the processors 130.

A CPU is a generic-purpose processor 130 that can perform not only general operations but also artificial intelligence operations, and it can effectively execute a complex program through a multilayer cache structure. A CPU is advantageous for a serial processing method that enables a systemic linking between the previous calculation result and the next calculation result through sequential calculations. Meanwhile, the generic-purpose processor 130 is not limited to the aforementioned examples excluding cases wherein it is specified as the aforementioned CPU.

A GPU is a processor 130 for mass operations such as a floating point operation used for graphic processing, etc., and it can perform mass operations in parallel by massively integrating cores. In particular, a GPU may be advantageous for a parallel processing method such as a convolution operation, etc. compared to a CPU. Also, a GPU may be used as a coprocessor 130 for supplementing the function of a CPU. Meanwhile, the processor 130 for mass operations is not limited to the aforementioned examples excluding cases wherein it is specified as the aforementioned GPU.

An NPU is a processor 130 specialized for an artificial intelligence operation using an artificial neural network, and it can implement each layer constituting an artificial neural network as hardware (e.g., silicon). Here, the NPU is designed to be specialized according to the required specification of a company, and thus it has a lower degree of freedom compared to a CPU or a GPU, but it can effectively process an artificial intelligence operation required by the company. Meanwhile, as the processor 130 specialized for an artificial intelligence operation, the NPU may be implemented in various forms such as a tensor processing unit (TPU), an intelligence processing unit (IPU), a vision processing unit (VPU), etc. Meanwhile, the artificial intelligence processor 130 is not limited to the aforementioned examples excluding cases wherein it is specified as the aforementioned NPU.

Also, the one or plurality of processors 130 may be implemented as a system on chip (SoC). Here, in the SoC, the memory 120, and a network interface such as a bus for data communication between the processor 130 and the memory 120, etc. may be further included other than the one or plurality of processors 130.

In case the plurality of processors 130 are included in the system on chip (SoC) included in the electronic device 100, the electronic device 100 may perform an operation related to artificial intelligence (e.g., an operation related to learning or inference of the artificial intelligence model) by using some processors 130 among the plurality of processors 130. For example, the electronic device 100 may perform an operation related to artificial intelligence by using at least one of a GPU, an NPU, a VPU, a TPU, or a hardware accelerator specified for artificial intelligence operations such as a convolution operation, a matrix product operation, etc. among the plurality of processors 130. However, this is merely an example, and the electronic device 100 can obviously process an operation related to artificial intelligence by using the generic-purpose processor 130 such as a CPU, etc.

Also, the electronic device 100 may perform operations related to artificial intelligence by using a multicore (e.g., a dual core, a quad core, etc.) included in the one processor 130. In particular, the electronic device 100 may perform artificial intelligence operations such as a convolution operation, a matrix product operation, etc. in parallel by using the multicore included in the processor 130.

The one or plurality of processors 130 perform control such that input data is processed according to pre-defined operation rules or an artificial intelligence model stored in the memory 120. The pre-defined operation rules or the artificial intelligence model are characterized in that they are made through learning.

Here, being made through learning means that a learning algorithm is applied to a plurality of learning data, and pre-defined operation rules or an artificial intelligence model having desired characteristics are thereby made. Such learning may be performed in a device itself wherein artificial intelligence is performed according to the disclosure, or performed through a separate server and/or system.

An artificial intelligence model may consist of a plurality of neural network layers. At least one layer has at least one weight value, and performs an operation of the layer through the operation result of the previous layer and at least one defined operation. As examples of a neural network, there are a convolutional neural network (CNN), a deep neural network (DNN), a recurrent neural network (RNN), a restricted Boltzmann machine (RBM), a deep belief network (DBN), a bidirectional recurrent deep neural network (BRDNN), deep Q-networks, and a Transformer, but the neural network in the disclosure is not limited to the aforementioned examples excluding specified cases.

A learning algorithm is a method of training a specific subject device (e.g., a robot) by using a plurality of learning data, and thereby making the specific subject device make a decision or make prediction by itself. As examples of learning algorithms, there are supervised learning, unsupervised learning, semi-supervised learning, or reinforcement learning, but learning algorithms in the disclosure are not limited to the aforementioned examples excluding specified cases.

Meanwhile, a storage medium that is readable by machines may be provided in the form of a non-transitory storage medium. Here, the term 'a non-transitory storage medium' only means that the device is a tangible device, and does not include a signal (e.g.: an electromagnetic wave), and the term does not distinguish a case wherein data is stored semi-permanently in a storage medium and a case wherein data is stored temporarily. For example, 'a non-transitory storage medium' may include a buffer wherein data is temporarily stored.

According to an embodiment, methods according to the various embodiments disclosed herein may be provided while being included in a computer program product. A computer program product refers to a product, and it can be traded between a seller and a buyer. A computer program product can be distributed in the form of a storage medium that is readable by machines (e.g.: compact disc read only memory (CD-ROM)), or distributed directly on-line (e.g.: download or upload) through an application store (e.g.: Play Store^{™}), or between two user devices (e.g.: smartphones). In the case of on-line distribution, at least a portion of a computer program product (e.g.: a downloadable app) may be stored in a storage medium readable by machines such as the server of the manufacturer, the server of the application store, and the memory of the relay server at least temporarily, or may be generated temporarily.

Also, each of the components (e.g.: a module or a program) according to the aforementioned various embodiments of the disclosure may consist of a singular object or a plurality of objects. In addition, among the aforementioned corresponding sub components, some sub components may be omitted, or other sub components may be further included in the various embodiments. Alternatively or additionally, some components (e.g.: a module or a program) may be integrated as an object, and perform functions that were performed by each of the components before integration identically or in a similar manner.

Further, operations performed by a module, a program, or other components according to the various embodiments may be executed sequentially, in parallel, repetitively, or heuristically. Or, at least some of the operations may be executed in a different order or omitted, or other operations may be added.

Meanwhile, the term "a part" or "a module" used in the disclosure may include a unit implemented as hardware, software, or firmware, and may be interchangeably used with, for example, terms such as a logic, a logical block, a component, or circuitry. In addition, "a part" or "a module" may be a component constituted as an integrated body or a minimum unit or a part thereof performing one or more functions. For example, a module may be constituted as an application-specific integrated circuit (ASIC).

Also, the various embodiments of the disclosure may be implemented as software including instructions stored in machine-readable storage media, which can be read by machines (e.g.: computers). The machines refer to devices that call instructions stored in a storage medium, and can operate according to the called instructions, and the devices may include an electronic device according to the aforementioned embodiments (e.g.: the electronic device 100).

In case an instruction is executed by a processor, the processor may perform a function corresponding to the instruction by itself, or by using other components under its control. An instruction may include a code that is generated or executed by a compiler or an interpreter.

It will be appreciated that various embodiments of the disclosure according to the claims and description in the specification can be realized in the form of hardware, software or a combination of hardware and software.

Any such software may be stored in non-transitory computer readable storage media. The non-transitory computer readable storage media store one or more computer programs (software modules), the one or more computer programs include computer-executable instructions that, when executed by one or more processors of an electronic device individually or collectively, cause the electronic device to perform a method of the disclosure.

Any such software may be stored in the form of volatile or non-volatile storage such as, for example, a storage device like read only memory (ROM), whether erasable or rewritable or not, or in the form of memory such as, for example, random access memory (RAM), memory chips, device or integrated circuits or on an optically or magnetically readable medium such as, for example, a compact disk (CD), digital versatile disc (DVD), magnetic disk or magnetic tape or the like. It will be appreciated that the storage devices and storage media are various embodiments of non-transitory machine-readable storage that are suitable for storing a computer program or computer programs comprising instructions that, when executed, implement various embodiments of the disclosure. Accordingly, various embodiments provide a program comprising code for implementing apparatus or a method as claimed in any one of the claims of this specification and a non-transitory machine-readable storage storing such a program.

While the disclosure has been shown and described with reference to various embodiments thereof, it will be understood by those skilled in the art that various changes inform and details may be made therein without departing from the spirit and scope of the disclosure as defined by the appended claims and their equivalents.

## Claims

1. An electronic device comprising:
a microphone;
memory storing one or more computer programs; and
one or more processors communicatively coupled to the microphone, and the memory,
wherein the one or more computer programs include computer-executable instructions that, when executed by the one or more processors individually or collectively, cause the electronic device to:
store registration information on breathing sounds of a plurality of users in the memory,
based on receiving an audio signal through the microphone, obtain information on a breathing sound of a user based on the audio signal,
compare the information on the breathing sound with the registration information,
identify at least one user corresponding to the information on the breathing sound among the plurality of users, and
based on identifying the at least one user, obtain an analysis result for sleeping states of each of the at least one user based on information corresponding to each of the at least one user in the information on the breathing sound.

2. The electronic device of claim 1, wherein the one or more computer programs further include computer-executable instructions that, when executed by the one or more processors individually or collectively, cause the electronic device to:
based on receiving the audio signal, identify a plurality of segments corresponding to the breathing sound of the user in the audio signal,
obtain a plurality of first embedding vectors corresponding to each of the plurality of segments, and
based on comparing each of the plurality of first embedding vectors with a plurality of second embedding vectors corresponding to the registration information, identify the at least one user.

3. The electronic device of claim 2, wherein the one or more computer programs further include computer-executable instructions that, when executed by the one or more processors individually or collectively, cause the electronic device to:
based on obtaining the plurality of first embedding vectors, identify distances between locations of each of the plurality of first embedding vectors and a centroid location of embedding vectors corresponding to a first user among the plurality of second embedding vectors in a latent space, and
based on the identified distances being smaller than a predetermined threshold distance, identify the first user as the at least one user.

4. The electronic device of claim 2, wherein the one or more computer programs further include computer-executable instructions that, when executed by the one or more processor individually or collectively, cause the electronic device to:
obtain information on the plurality of segments by inputting the audio signal into a first neural network model trained to distinguish a breathing sound of a user included in an audio signal,
obtain the plurality of first embedding vectors by inputting the plurality of segments into a second neural network model trained to convert an input segment into an embedding vector, and
obtain the analysis result by inputting the information corresponding to each of the at least one user into a third neural network model trained to identify a sleeping state of a user corresponding to a breathing sound.

5. The electronic device of claim 1, wherein the analysis result comprises at least one of information on whether the at least one user is sleeping, information on sleep quality of the at least one user, or information on health of the at least one user.

6. The electronic device of claim 1, further comprising:
a transceiver,
wherein the one or more computer programs further include computer-executable instructions that, when executed by the one or more processors individually or collectively, cause the electronic device to:
based on the analysis result indicating that the at least one user is sleeping, obtain a control signal for controlling an external device, and
transmit the control signal to the external device.

7. The electronic device of claim 1, wherein the one or more computer programs further include computer-executable instructions that, when executed by the one or more processors individually or collectively, cause the electronic device to:
based on identifying the at least one user, update the registration information based on the information on the breathing sound.

8. The electronic device of claim 1, further comprising:
a display,
wherein the one or more computer programs further include computer-executable instructions that, when executed by the one or more processors individually or collectively, cause the electronic device to:
based on the at least one user not being identified, display a user interface, and
based on receiving a user input for registering the information on the breathing sound through the user interface, add the information on the breathing sound to the registration information.

9. The electronic device of claim 1, further comprising:
a sensor,
wherein the one or more computer programs further include computer-executable instructions that, when executed by the one or more processors individually or collectively, cause the electronic device to:
based on identifying the at least one user, obtain biometric information of the at least one user through the sensor, and
obtain the analysis result for the sleeping states of each of the at least one user based on the information corresponding to each of the at least one user and the biometric information.

10. A method performed by an electronic device, the method comprising:
storing registration information on breathing sounds of a plurality of users;
based on receiving an audio signal, obtaining information on a breathing sound of a user based on the audio signal;
comparing the information on the breathing sound with the registration information on breathing sounds of a plurality of users;
identifying at least one user corresponding to the information on the breathing sound among the plurality of users; and
based on identifying the at least one user, obtaining an analysis result for sleeping states of each of the at least one user based on information corresponding to each of the at least one user in the information on the breathing sound.

11. The method of claim 10,
wherein the obtaining of the information on the breathing sound comprises:
based on receiving the audio signal, identifying a plurality of segments corresponding to the breathing sound of the user in the audio signal, and
obtaining a plurality of first embedding vectors corresponding to each of the plurality of segments, and
wherein the identifying of the at least one user comprises:
based on comparing each of the plurality of first embedding vectors with a plurality of second embedding vectors corresponding to the registration information, identifying the at least one user.

12. The method of claim 11, wherein the identifying of the at least one user further comprises:
based on obtaining the plurality of first embedding vectors, identifying distances between locations of each of the plurality of first embedding vectors and a centroid location of embedding vectors corresponding to a first user among the plurality of second embedding vectors in a latent space; and
based on the identified distances being smaller than a predetermined threshold distance, identifying the first user as the at least one user.

13. The method of claim 11,
wherein the obtaining of the information on the breathing sound comprises:
obtaining information on the plurality of segments by inputting the audio signal into a first neural network model trained to distinguish a breathing sound of a user included in an audio signal, and
obtaining the plurality of first embedding vectors by inputting the plurality of segments into a second neural network model trained to convert an input segment into an embedding vector, and
wherein the obtaining of the analysis result comprises:
obtaining the analysis result by inputting the information corresponding to each of the at least one user into a third neural network model trained to identify a sleeping state of a user corresponding to a breathing sound.

14. The method of claim 10, wherein the analysis result comprises at least one of information on whether the at least one user is sleeping, information on sleep quality of the at least one user, or information on health of the at least one user.

15. The method of claim 10, further comprising:
based on the analysis result indicating that the at least one user is sleeping, obtaining a control signal for controlling an external device; and
transmitting the control signal to the external device.
